# EUROPEAN PATENT SPECIFICATION

(11) **EP 3 466 377 B1**
(45) Date of publication and mention of the grant of the patent: **05.07.2023**
(21) Application number: 17806900.1
(22) Date of filing: 12.05.2017
(51) Int. Cl.: A61F 2/954, A61M 25/00, A61M 25/01

(54) **STENT INSERTION APPARATUS COMPRISING GUIDE MEMBER HAVING FIXED PART**
STENTEINSETZVORRICHTUNG MIT FÜHRUNGSELEMENT MIT FESTEM TEIL
APPAREIL D'INSERTION D'ENDOPROTHÈSE COMPRENANT UN ÉLÉMENT DE GUIDAGE PRÉSENTANT UNE PARTIE FIXE

(30) Priority: 30.05.2016 KR 20160066493
(43) Date of publication of application: 10.04.2019
(73) Proprietor: S&G Biotech, Inc, Seongnam-si, Gyeonggi-do 13219 (KR)
(72) Inventor: SUKO, Adiarto, Joglo Jakarta Barat 11640 (ID); KANG, Sung Kwon, Yongin-si Gyeonggi-do 16876 (KR)
(74) Representative: Ter Meer Steinmeister & Partner
(86) International application number: PCT/KR2017/004917
(87) International publication number: WO 2017/209407

(56) References cited:
- EP-A2- 2 517 671
- EP-B1- 1 351 624
- JP-A- 2010 520 026
- KR-A- 20130 140 961
- KR-B1- 100 822 045
- KR-B1- 101 080 137
- KR-B1- 101 709 601
- US-A1- 2008 221 656
- US-A1- 2013 030 514

## Description

### [Technical Field]

The present invention relates to a stent insertion apparatus with which a stent may be installed in a branched area of stenosis in addition to a main area of stenosis when stenosis occurs at a blood vessel in a human body, and more particularly, to a stent insertion apparatus in which a fixing part configured to fix a guide tube for installing a stent in a branched area of stenosis is formed at a guide member inserted into a main area of stenosis to guide the stent to an installation position of the stent.

### [Background Art]

In a case in which stenosis occurs due to sediment or the like in an organ such as a blood vessel, or esophagus in which a certain inner diameter thereof in needs to be secured in a human body, a stent insertion apparatus which inserts a stent to artificially expand an area of stenosis is generally used.

However, in a case in which the branched area of stenosis branches from and is adjacent to the area of stenosis, there is a problem in that it is difficult to insert a separate stent into the branched area of stenosis using such a conventional stent insertion apparatus. In addition, in a case in which the stenosis is caused by sediment or the like, a phenomenon in which the sediment is pushed into the branched area of stenosis frequently occurs while the stent is installed in the main area of stenosis, which becomes a cause of increasing difficulty in a stent insertion operation. This problem has already been recognized in the prior art.

In order to solve it, US 2008/221656 A1 proposes an endovascular delivery device that has a portion which is formed from a radiopaque material, wherein the portion has a selected transverse profile such as a notch so that the position and orientation of the endovascular delivery device can be observed by radiographic means during an endovascular procedure.

Another prior art document, US 2013/030514 A1, describes a stent graft delivery device having an elongated extension piece extending from the proximal end of the introducer portion, wherein the elongate extension piece is separable from the introducer portion and wherein a plurality of auxiliary guide wires extend from through the stent graft delivery device and through the elongate extension piece.

To solve such a problem, a method is proposed in which a guide tube for guiding a stent to be installed in a branched area of stenosis is inserted at the time when the stent is inserted into a main area of stenosis using a stent insertion apparatus.

The guide tube may be installed in the branched area of stenosis while the stent is inserted into the main area of stenosis, and accordingly, a stent to be installed in the branched area of stenosis is inserted using another stent insertion apparatus.

However, the above-described method has a problem in that it is technically very difficult to position the guide tube at the branched area of stenosis during an operation. In addition, a case in which the guide tube was separated from the stent insertion apparatus occurred during the operation, and in this case, since the stent insertion apparatus inserted into a human body has to be collected to fix the guide tube again, there is a problem in that severe time loss occurs, which can cause medical accidents.

Accordingly, a method for solving such a problem is required.

### [Disclosure] [Technical Problem]

The present invention is directed to preventing a phenomenon in which a guide tube into which a guide wire for guiding a stent to a branched area of stenosis is inserted is drawn and separated during an operation for installing stents in a main area of stenosis and a branched area of stenosis.

Objectives of the present invention are not limited thereto, and other objectives which are not mentioned above will be obviously understood by those skilled in the art from the following description.

### [Technical Solution]

One aspect of the present invention provides a stent insertion apparatus according to claim 1. This apparatus includes, amongst others, a guide member in which a fixing part is formed, which includes a guide member configured to guide movement of a first stent inserted into a human body along a first guide wire inserted into a main area of stenosis and configured to be installed in the main area of stenosis, wherein the guide member includes a fixing part configured to fix a leading end portion of a guide tube into which a second guide wire for installing a second stent is inserted, the second stent being inserted into a branched area of stenosis, which branches from the main area of stenosis, to be installed in the branched area of stenosis.

In addition, the fixing part may include an insertion hole formed in a longitudinal direction of the guide member such that a leading end portion of the guide tube is inserted into the insertion hole.

In addition, a depth of the insertion hole may be greater than a diameter of the guide tube.

In addition, as the depth of the insertion hole increases, a width thereof may gradually increase.

In addition, a width of an upper portion of the insertion hole may be less than the diameter of the guide tube.

In addition, a pair of fixed protrusions spaced by a distance which is less than the diameter of the guide tube from each other may be formed on both sides of the upper portion of the insertion hole.

In addition, a cover configured to shield a part of the insertion hole may be formed at the upper portion of the insertion hole.

In addition, a lateral cross sectional area of at least a part of the insertion hole may be less than that of the guide tube.

In addition, a plurality of guide tubes are provided, and the number of fixing parts may be formed to correspond to the number of the guide tubes.

### [Advantageous Effects]

A stent insertion apparatus including a guide member in which a fixing part is formed according to the present invention for solving the above-described problem has the following effects.

First, since a fixing part for fixing a guide tube to a guide member configured to guide a first stent to a main area of stenosis is formed, there is an advantage in that a phenomenon in which the guide tube is drawn and separated can be prevented during an operation.

Second, there is an advantage in that the guide tube can be accurately positioned at a desired branched area of stenosis by adjusting a fixing area of the guide tube during the operation.

Third, there is an advantage in that an additional operation process is not necessary during a process of inserting the guide tube into the branched area of stenosis.

Effects of the present invention are not limited to the above-described effects, and effects which have not been described above will be clearly understood by those skilled in the art through the append claims.

### [Description of Drawings]

FIG. 1 is a view illustrating a stent insertion apparatus according to a first embodiment of the present invention.
FIG. 2 is a view illustrating main parts of the stent insertion apparatus according to the first embodiment of the present invention.
FIG. 3 is a cross-sectional view illustrating the main parts of the stent insertion apparatus according to the first embodiment of the present invention.
FIGS. 4 and 5 are views illustrating a guide member of the stent insertion apparatus according to the first embodiment of the present invention.
FIG. 6 is a view illustrating a state in which a first guide wire is inserted into a main area of stenosis during an operation process using the stent insertion apparatus according to the first embodiment of the present invention.
FIG. 7 is a view illustrating a state in which a first outer tube is inserted into the main area of stenosis along the first guide wire during the operation process using the stent insertion apparatus according to the first embodiment of the present invention.
FIGS. 8 and 9 are views illustrating a state in which a first inner tube moves forward to discharge a part of a first stent during the operation process using the stent insertion apparatus according to the first embodiment of the present invention.
FIG. 10 is a view illustrating a state in which a guide tube is inserted into a branched area of stenosis during the operation process using the stent insertion apparatus according to the first embodiment of the present invention.
FIGS. 11 and 12 are views illustrating a state in which a fixing wire is removed to expand the first stent in the main area of stenosis a during the operation process using the stent insertion apparatus according to the first embodiment of the present invention.
FIG. 13 is a view illustrating a state in which the remaining components except for the first stent and the second guide wire are removed during the operation process using the stent insertion apparatus according to the first embodiment of the present invention.
FIGS. 14 and 15 are views illustrating a state in which a second outer tube is positioned in the branched area of stenosis during the operation process using the stent insertion apparatus according to the first embodiment of the present invention.
FIG. 16 is a view illustrating a state in which a second inner tube moves forward to discharge a second stent during the operation process using the stent insertion apparatus according to the first embodiment of the present invention.
FIG. 17 is a view illustrating a state in which the fixing wire is removed to expand the second stent in the branched area of stenosis during the operation process using the stent insertion apparatus according to the first embodiment of the present invention.
FIG. 18 is a view illustrating a state in which the second stent is installed in each branched area of stenosis during the operation process using the stent insertion apparatus according to the first embodiment of the present invention.
FIG. 19 is a view illustrating a guide member of a stent insertion apparatus according to a second embodiment of the present invention.
FIG. 20 is a view illustrating a guide member of a stent insertion apparatus according to a third embodiment of the present invention.
FIG. 21 is a view illustrating a guide member of a stent insertion apparatus according to a fourth embodiment of the present invention.
FIGS. 22 and 23 are views illustrating a state in which guide tubes are inserted into branched areas of stenosis during an operation process using the stent insertion apparatus according to the fourth embodiment of the present invention.
FIG. 24 is a view illustrating a guide member of a stent insertion apparatus according to a fifth embodiment of the present invention.

### [Modes of the Invention]

Hereinafter, exemplary embodiments of the present invention that may specifically realize the objectives of the present invention will be described with reference to the accompanying drawings. While the present embodiments are described, components that are the same are referred to by the same reference numerals in the specification and additional descriptions will be omitted.

The present invention relates to a stent insertion apparatus as defined in claim 1 and includes a guide member configured to guide movement of a first stent inserted into a human body along a first guide wire inserted into a main area of stenosis so as to be installed in the main area of stenosis, and the guide member includes a fixing part which fixes a guide tube into which a second guide wire for installing a second stent is inserted, wherein the second stent is inserted into a branched area of stenosis, which branches from the main area of stenosis, to be installed in the branched area of stenosis.

Here, the fixing part may be formed in various shapes, and there may also be a variety of fixing methods thereof. Accordingly, the present invention may prevent a phenomenon in which the guide tube is drawn and separated and also adjust a fixing position of the guide tube to accurately position the guide tube at the desired branched area of stenosis during an operation.

Hereinafter, the present invention will be described in detail with reference to detailed embodiments according to the above-described present invention.

A stent insertion apparatus according to the present invention includes a first stent insertion unit and a second stent insertion unit, and each of the first stent insertion unit and the second stent insertion unit includes an insertion part and an auxiliary part.

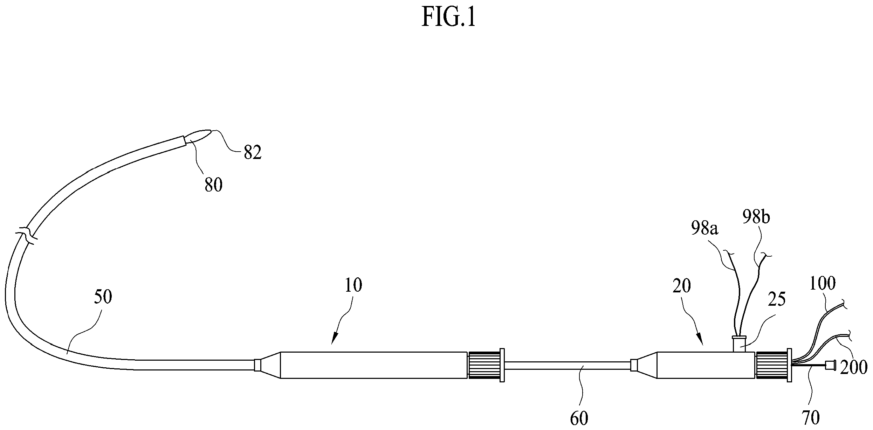

First, referring to FIG.1, FIG. 1 shows an overall shape of a stent insertion apparatus according to a first embodiment of the present invention. Only the first stent insertion unit is illustrated in the drawing, and a second stent insertion unit will be described with reference to another drawing below.

As illustrated in the drawing, the first stent insertion unit according to the present invention includes a first insertion part 10 and a first auxiliary part 20. A first outer tube 50 extends from the first insertion part 10, and an inner tube 60 extends from the first auxiliary part 20. In addition, the first inner tube 60 is inserted into the first insertion part 10 to be inserted into an inner space of the first outer tube 50.

In addition, the first insertion part 10 and first auxiliary part 20 are operated to slide the first inner tube 60 forward or backward in the first outer tube 50. Specifically, in a case in which the first insertion part 10 and the first auxiliary part 20 are operated such that a distance therebetween decreases, the first inner tube 60 moves forward in the first outer tube 50, and the first insertion part 10 and the first auxiliary part 20 are operated such that, in a case in which the distance increases, the first inner tube 60 moves backward in the first outer tube 50.

Meanwhile, the first insertion part 10 and the first auxiliary part 20 may have various shapes, but in the first embodiment, have long bodies so that a user may easily grip the bodies. In addition, the first outer tube 50 and the first inner tube 60 respectively extend in front of the first insertion part 10 and the first auxiliary part 20, and the first inner tube 60 is inserted into the first insertion part 10 from a behind thereof.

In addition, a second guide wire 2 in addition to the first inner tube 60 is inserted into the first insertion part 10, and a first central tube 70 is inserted into the first auxiliary part 20 to pass through an inner space of the first inner tube 60, which will be described below.

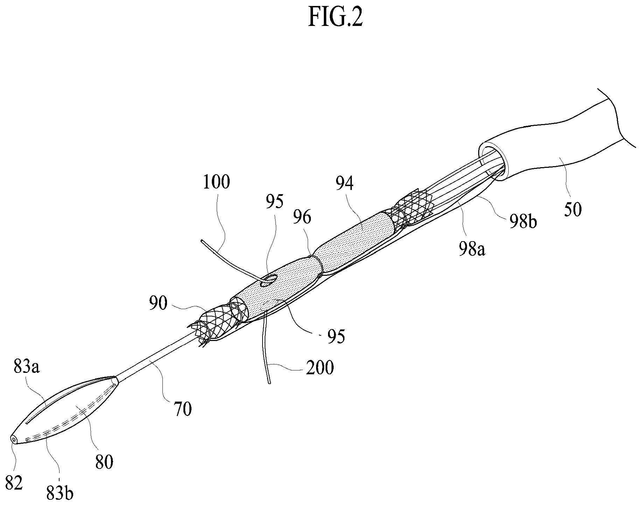

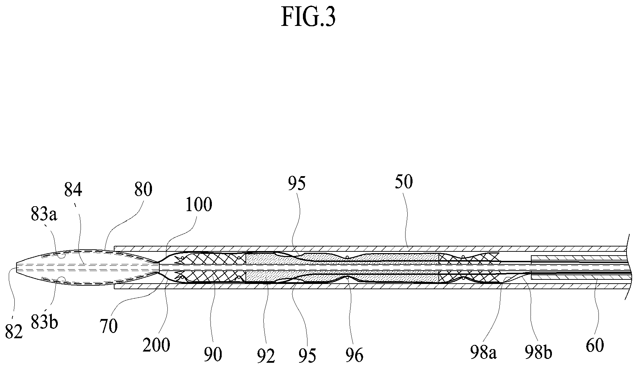

Hereinafter, the first stent insertion unit will be described with reference to FIGS. 2 and 3 in order to describe the first stent insertion unit in more detail. A leading end portion of the first outer tube 50 is illustrated in FIG. 2, and a cross-sectional view of the leading end portion of the first outer tube 50 is illustrated in FIG. 3.

First, referring to the cross-sectional view of FIG. 3, it can be seen that the first inner tube 60 is inserted into the inner space of the first outer tube 50, and the first central tube 70 passes through the inner space of the first inner tube 60 as described above.

In addition, a guide member 80 is provided at a leading end of the first central tube 70 and guides a first guide wire to be inserted into a guide hole 82 of an entrance thereof to guide the first outer tube 50 to a path of the first outer tube.

In addition, a connecting hole 84 connected to the guide hole 82 is formed in the guide member 80. The connecting hole 84 may also be connected to an inner space of the first central tube 70, or the first central tube 70 may also be inserted into the guide member 80.

In the case of the present embodiment, the first central tube 70 is inserted into the guide member 80 as illustrated in FIG. 3. As a result, the first guide wire may be inserted into the guide hole 82 of the guide member 80 along the inner space of the first central tube 70.

Here, a thickness of the guide member 80 is greater than a diameter of the first outer tube 50 so that the guide member 80 cannot be inserted into the first outer tube 50 and protrudes outward. Accordingly, in a case in which the first outer tube 50 is inserted into a human body, a resistance generated due to a body fluid decreases and a position of the guide member 80 is fixed to the leading end of the first outer tube 50 so that the guide member 80 performs its role.

Meanwhile, the guide member 80 and the first inner tube 60 may be positioned to be spaced a predetermined distance from each other to secure a space in which the first stent 90 may be accommodated. Specifically, the first stent 90 is positioned at the space by which the guide member 80 and the first inner tube 60 are spaced apart from each other in a state in which the first stent 90 surrounds a circumference of the first central tube 70.

Accordingly, in a case in which the first insertion part 10 and the first auxiliary part 20 are operated such that the distance between the first insertion part 10 and the first auxiliary part 20 decreases, the first inner tube 60 moves forward in the first outer tube 50 to form a structure in which the first inner tube 60 pushes the first stent 90 outward. That is, the first inner tube 60 may move forward to push the first stent 90 and the guide member 80 outward from the first outer tube 50 and discharge the first stent 90.

As described above, in the case in which the first stent 90 is discharged from the first outer tube 50, a diameter of the first stent 90 increases to support an inner wall of an area in which stenosis occurs and to secure a diameter of the area of stenosis which is greater than or equal to a predetermined area. Here, the first stent 90 may also be expanded by another apparatus such as a balloon catheter or by itself.

In the first embodiment, the first stent 90 may be expanded by itself, and the first stent 90 may be implemented by being formed of a shape-memory alloy or formed to have elasticity.

In addition, in the case of the present embodiment, the main area of stenosis is an abdominal aorta, and the branched area of stenosis is a renal artery which branches from the abdominal aorta. Accordingly, an artificial blood vessel 94 surrounds an outer side of the first stent 90 in the present embodiment, and since technology about the artificial blood vessel 94 is known and commonly used, a detailed description thereof will be omitted.

In addition, one or more expansion preventing members 96 configured to prevent expansion of the first stent 90 may be provided at a circumference of the first stent 90. Accordingly, even in a case in which the first stent 90 is discharged outward from the first outer tube 50, the expansion preventing members 96 serve to prevent expansion of the first stent 90.

In a case in which an expansion preventing member 96 is not provided, the first stent 90 starts to expand at a moment at which the first stent 90 is discharged from the first outer tube 50, and in a case in which the first stent 90 is completely discharged, the first stent 90 comes into contact with the inner wall of the area of stenosis, and thus there is a problem in that it is difficult to correct an installation position thereof. However, in the case in which the expansion preventing members 96 are provided, there is an advantage in that the first stent 90 may be discharged first, a position thereof may be precisely adjusted, and an expansion operation may be performed.

Particularly, in the present embodiment, the expansion preventing member 96 is formed in a ring shape such that an elastic force is applied in a direction in which the expansion preventing member 96 having the ring shape is stretched out, and lower end portions thereof are not connected to each other and have a hook shape. In addition, the expansion preventing member 96 is fixed not to be stretched out by fixing wires 98a and 98b inserted into the expansion preventing member 96 having the hook shape.

In addition, the plurality of expansion preventing members 96 are provided, and particularly, a total of two expansion preventing members 96 are provided in front of the artificial blood vessel 94. Here, a second fixing wire 98b is inserted into the expansion preventing member which is closer to the artificial blood vessel 94 among the expansion preventing members positioned in front of the artificial blood vessel 94, and the first fixing wire 98a is inserted into the remaining expansion preventing members.

That is, the expansion preventing member which is closer to the artificial blood vessel 94 among the expansion preventing members disposed in front of the artificial blood vessel 94 may be expanded independently from the other expansion preventing members. This will be described below.

In addition, the fixing wires 98a and 98b extend to protrude outward from the first auxiliary part 20 through the first inner tube 60 as illustrated in FIG. 1. A protruding member 25 illustrated in FIG. 1 is provided to expose the fixing wires 98a and 98b, and in a case in which the fixing wires 98a and 98b exposed through the protruding member 25 are drawn from the first auxiliary part 20, the fixing wires 98a and 98b may move backward to be separated from the expansion preventing members 96 illustrated in FIG. 2 and expand the first stent 90.

That is, the expansion preventing members 96 and the fixing wires 98a and 98b operate in conjunction with each other to serve to delay expansion of the first stent 90, and thus a user may perform a more precise operation.

In addition, the expansion preventing members 96 may prevent expansion of the first stent 90 using various structures and methods in addition to the method used in the first embodiment.

Meanwhile, a pair of guide tubes 100 and 200 for operating branched areas of stenosis are inserted into the first insertion part 10 according to the present embodiment as illustrated in FIG. 1. The guide tubes 100 and 200 are guide tubes for guiding second stents for expanding branched areas of stenosis later and have hollows formed therein such that second guide wires, which will be described below, are inserted into the hollows.

The pair of guide tubes 100 and 200 are provided because branched areas of stenosis which branch from the abdominal aorta are a pair of renal arteries in the present embodiment. That is, the guide tubes 100 and 200 may be positioned at the different renal arteries.

In addition, the guide tubes 100 and 200 extend between the first outer tube 50 and the first inner tube 60, and the opposite sides thereof are fixed to the first stent 90 as illustrated in FIG. 2. That is, in a case in which the first stent 90 is discharged from the first outer tube 50, the guide tubes 100 and 200 may exit to the outside of the first outer tube 50 with the first stent 90.

Moreover, through holes 95 are formed in the artificial blood vessel 94 such that the guide tubes 100 and 200 pass through the through holes 95 to be fixed.

In addition, in the first embodiment, the guide tubes 100 and 200 are inserted into the first auxiliary part 20 from a behind thereof as illustrated in FIG. 1, but are not limited thereto, and may be inserted therein from various positions.

Meanwhile, the guide member 80 includes a fixing part configured to fix the guide tubes 100 and 200 as described above. The fixing part prevents a phenomenon in which the guide tubes 100 and 200 are detached and separated from the first stent 90 and easily guides the guide tubes 100 and 200 to the branched areas of stenosis during the operation process.

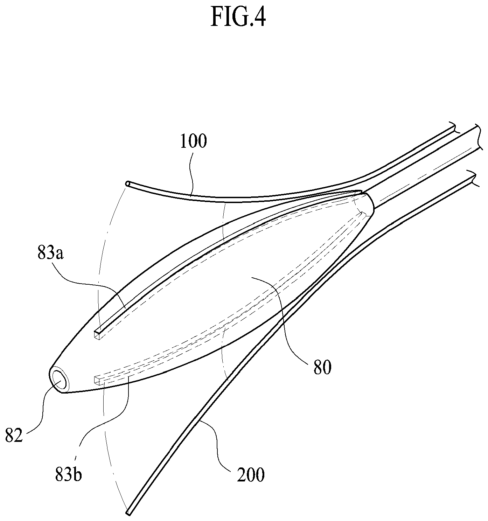

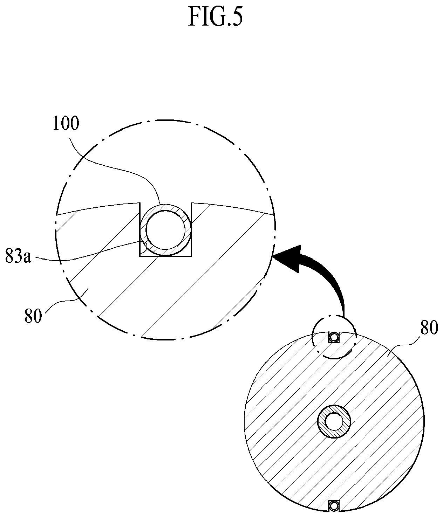

Particularly, the fixing part includes insertion holes 83a and 83b formed in a longitudinal direction of the guide member 80 such that leading end portions of the guide tubes 100 and 200 are inserted into the insertion holes 83a and 83b according to the present embodiment as illustrated in FIGS. 3 to 5. That is, the guide tubes 100 and 200 pass through the through holes 95 of the first stent 90, and the leading end portions of the guide tubes 100 and 200 are respectively inserted into the insertion holes 83a and 83b.

Here, depths of the insertion holes 83a and 83b may be formed to be larger than diameters of the guide tubes 100 and 200, and accordingly, the guide tubes 100 and 200 do not protrude outward from a circumference of the guide member 80.

Accordingly, since the guide tubes 100 and 200 are fixed in the insertion holes 83a and 83b, a phenomenon in which the guide tubes 100 and 200 are drawn and separated from the insertion holes 83a and 83b due to resistance may be prevented during the operation process.

In addition, the number of fixing parts may correspond to the number of the guide tubes 100 and 200.

The description about the first stent insertion unit has been completed above, and a method of operating in an area at which stenosis occurs in a human body will be described in detail. In addition, for example, areas in which the stenosis occurs are an abdominal aorta and a pair of renal arteries which branch from the abdominal aorta as described above.

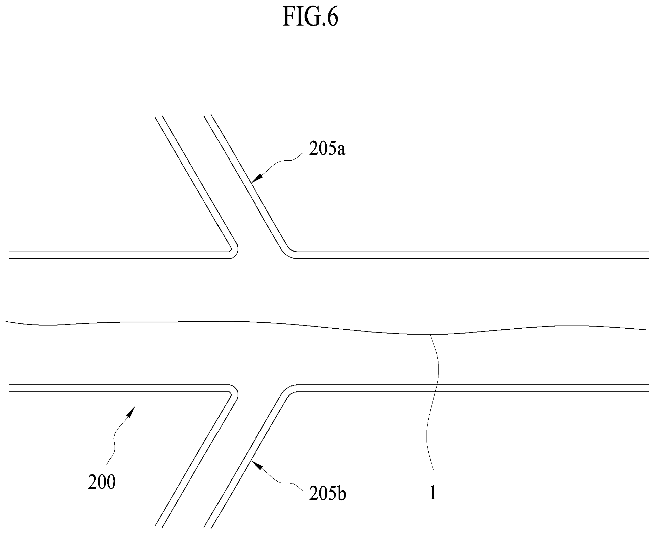

First, as illustrated in FIG. 6, a first guide wire 1 is inserted into an abdominal aorta 300. This process is a preceding process before inserting the stent insertion apparatus into the abdominal aorta 300, and the first guide wire 1 is inserted into the abdominal aorta 300 in advance.

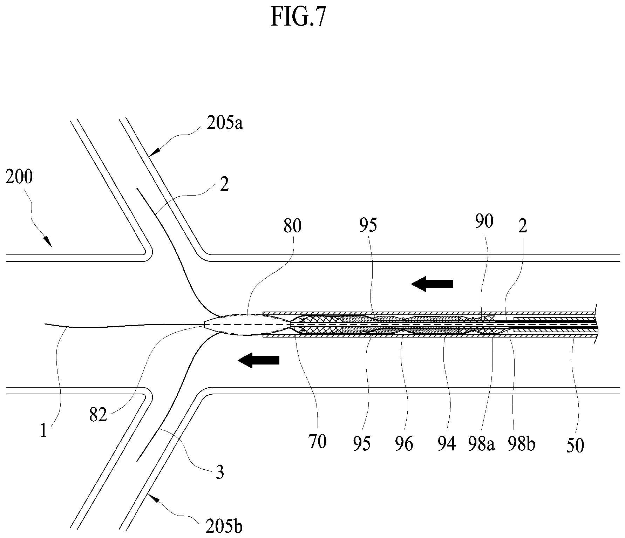

Next, as illustrated in FIG. 7, the first guide wire 1 is inserted into the guide hole 82 formed in the guide member 80 of the first stent insertion unit, the first guide wire 1 passes through the central tube 70, and the first outer tube 50 is positioned in the abdominal aorta 300 along the first guide wire 1. Accordingly, it can be seen that the leading end portion of the first outer tube 50 is positioned in the abdominal aorta 300 along a path of the first guide wire 1.

In addition, the second guide wires 2 and 3 are respectively inserted into the guide tubes 100 and 200 fixed to the guide member 80 and exposed forward from the guide tubes 100 and 200, and the second guide wires 2 and 3 are positioned in a state in which the second guide wires 2 and 3 are inserted into renal arteries 305a and 305b which branch from the abdominal aorta 300 during this process.

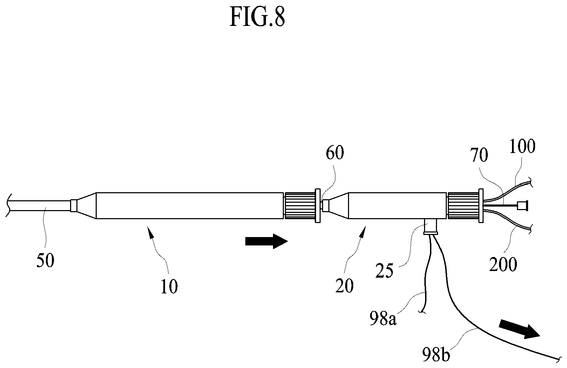

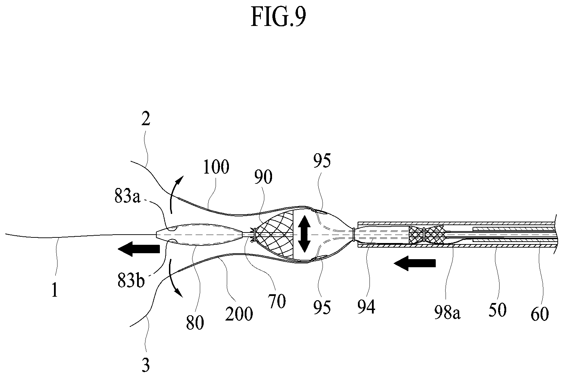

Next, as illustrated in FIGS. 8 and 9, the first stent insertion unit is operated to discharge a part of the first stent 90 from the first inner tube 60. Specifically, the first insertion part 10 and the first auxiliary part 20 are operated such that a distance therebetween decreases, and thus the first inner tube 60 moves forward in the first outer tube 50 to push the first stent 90 outward during the present step,.

Here, in order to perform the above-described operation, the first auxiliary part 20 may move forward in a state in which the first insertion part 10 is fixed, or the first insertion part 10 may move backward in a state in which the first auxiliary part 20 is fixed. That is, the operation for discharging the first stent 90 is performed by relative movements of the first insertion part 10 and the first auxiliary part 20, and thus an operator may operate the first insertion part 10 and the first auxiliary part 20 according to convenience thereof.

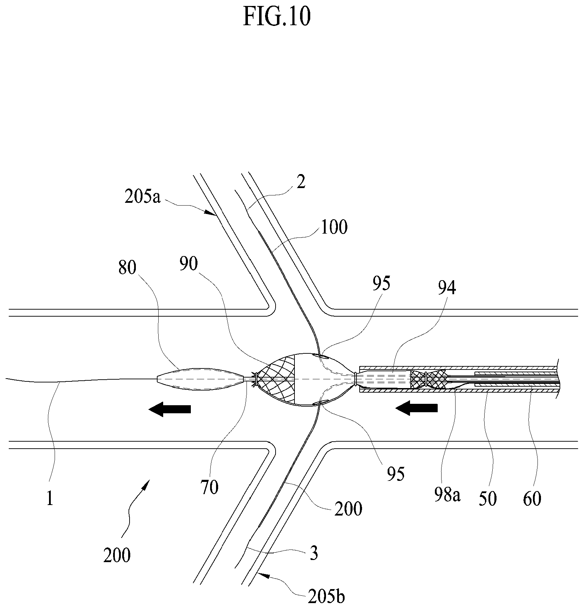

In addition, in this process, the pair of guide tubes 100 and 200 inserted into the insertion holes 83a and 83b of the guide member 80 are separated from the insertion holes 83a and 83b as a discharged part of the first stent 90 expands and are positioned at the renal arteries 305a and 305b as illustrated in FIG. 10.

As described above, in the present invention, since fixing positions of the guide tubes 100 and 200 are adjusted in advance, the guide tubes may be accurately positioned at the desired branched areas of stenosis, and since a separate operation process of inserting the guide tubes 100 and 200 into the branched areas of stenosis is also not required, the operation may be simply performed.

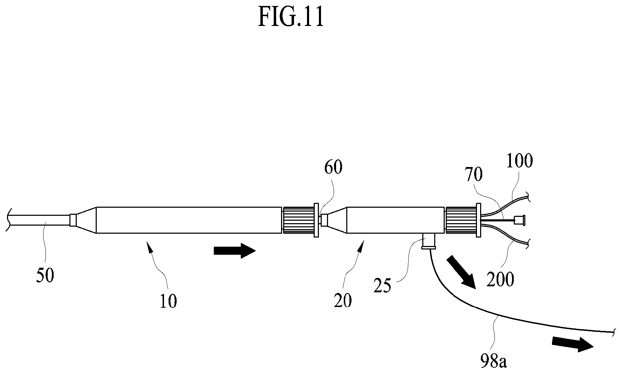

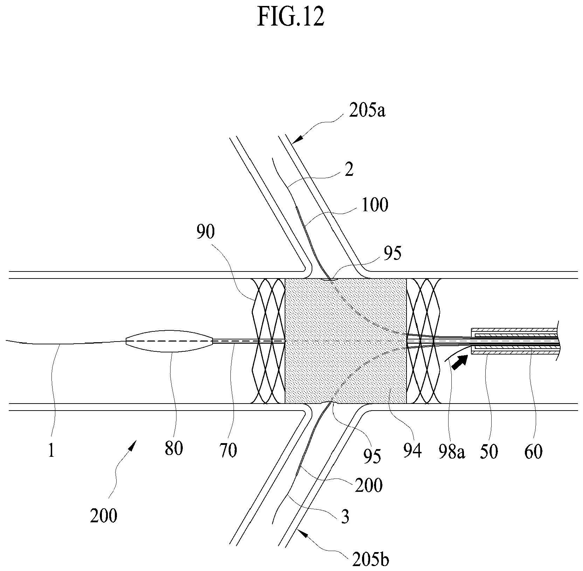

Next, as illustrated in FIGS. 11 and 12, a process of expanding the entire first stent 90 positioned in the abdominal aorta 300 is performed.

The present process may be performed by completely discharging the first stent 90 from the first inner tube 60, removing the first fixing wire 98a to stretch the expansion preventing member 96 out as illustrated in FIG. 11, and expanding the first stent 90 as illustrated in FIG. 12.

Through the above-described operation, the first stent 90 is completely installed in the abdominal aorta 300, and then an operation for installing the second stents in the renal arteries 305a and 305b is performed.

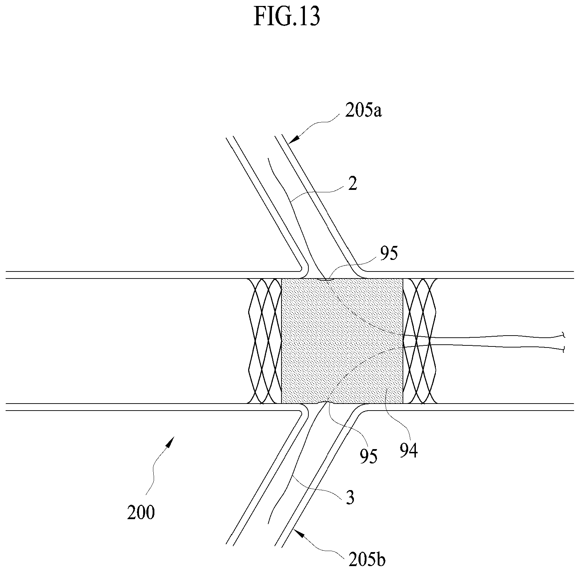

In addition, the remaining components except for the first stent 90 and the second guide wires 2 and 3 are removed from the abdominal aorta 300 in order to install the second stents as illustrated in FIG. 13. This is done so that the second stent insertion unit may be inserted later, which will be described in the following step.

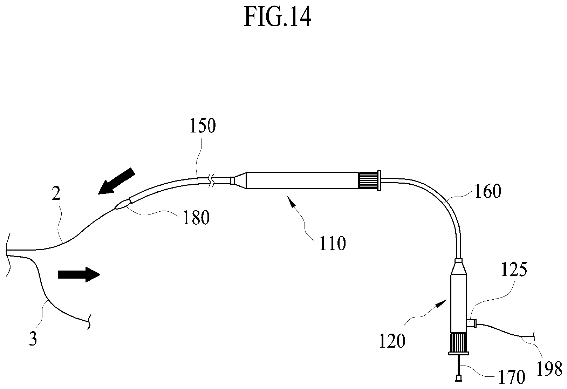

Next, as illustrated in FIG. 14, a second outer tube 150 of the second stent insertion unit is inserted through a rear end of any one of the pair of second guide wires 2 and 3 exposed through a rear of the first insertion part 10. Here, the second stent insertion unit is a unit for installing the second stents in the branched areas of stenosis, and an entire configuration and operation method thereof are the same as those of the first stent insertion unit.

That is, the second stent insertion unit includes a second insertion part 110 and a second auxiliary part 120, a second inner tube 160 is inserted into a second outer tube 150, and a second central tube 170 passes through the second inner tube 160 like the first stent insertion unit. In addition, the other detailed components of the second stent insertion unit are the same as those of the first stent insertion unit.

As a result, the second guide wire 2 is inserted into the second central tube 170, and the second outer tube 150 is pushed into and along the second guide wire 2 to be positioned in the renal artery 205a of one side thereof. That is, since the second guide wire 2 was inserted into the renal artery 205a by the previous operation, the second outer tube 150 may be positioned in the renal artery 205a along the second guide wire 2.

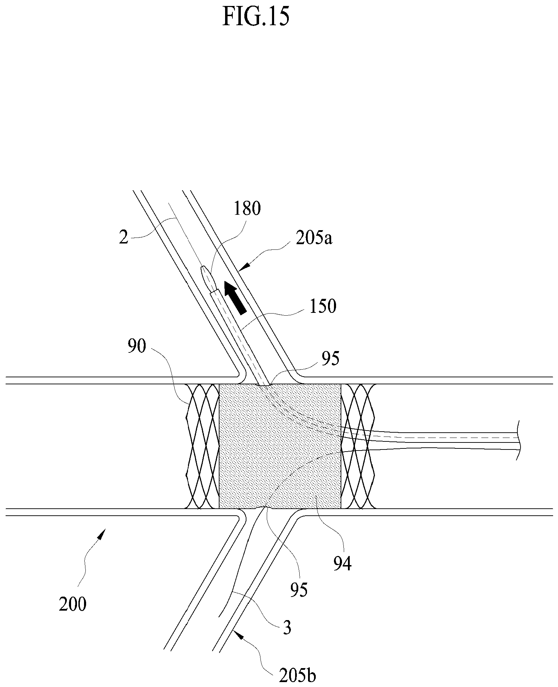

Referring to FIG. 15, it can be seen that the second outer tube 150 is positioned in the renal artery 205a of one side thereof through the through hole 95 formed in the artificial blood vessel 94 of the first stent 90.

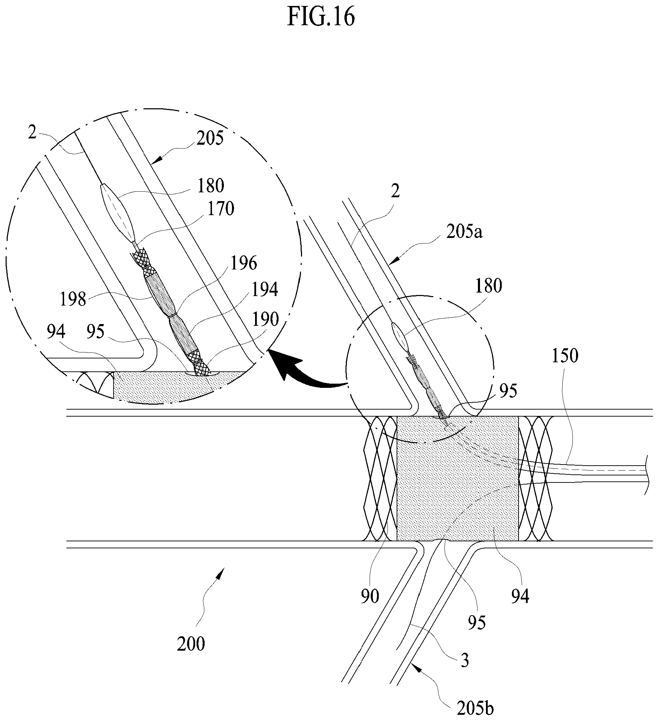

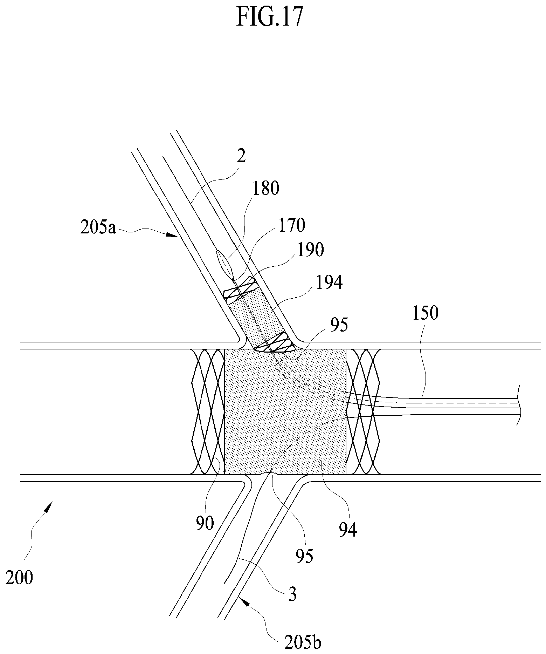

A process of expanding a second stent 190 is performed in an order which is the same as that of the expanding of the first stent 90. That is, the second stent insertion unit is operated to move the second inner tube 160 forward, discharge the second stent 190, and remove the fixing wire 198 to install the second stent 190 in the renal artery 205a, and the processes thereof are illustrated in FIGS. 16 and 17.

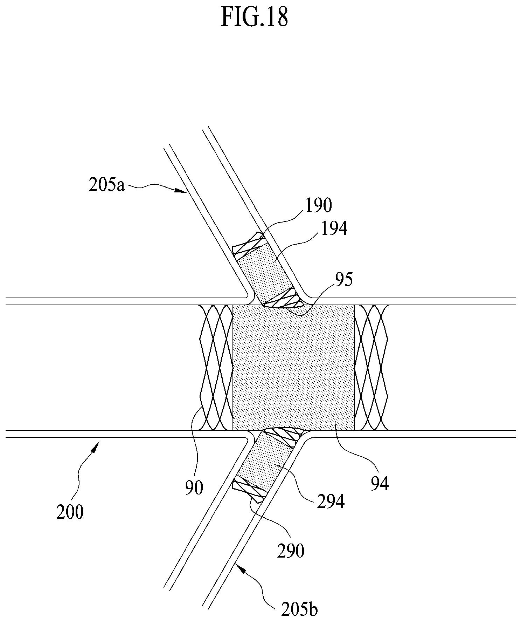

In addition, a process which is the same as the above-described process is performed for the renal artery 205b of the other side thereof to install a pair of second stents 190 and 290 including artificial blood vessels 194 and 294 at renal arteries 305a and 305b as illustrated in FIG. 18, and all of the processes are performed to completely install the first stent 90 and the second stents 190 and 290 in the abdominal aorta 300 and the renal arteries 305a and 305b. Next, the remaining components except for the installed stents 90 and 190 are removed to complete the operation.

The stent insertion apparatus according to the first embodiment of the present invention and the method of operating using the stent insertion apparatus have been described above. Hereinafter, other embodiments of the present invention will be described.

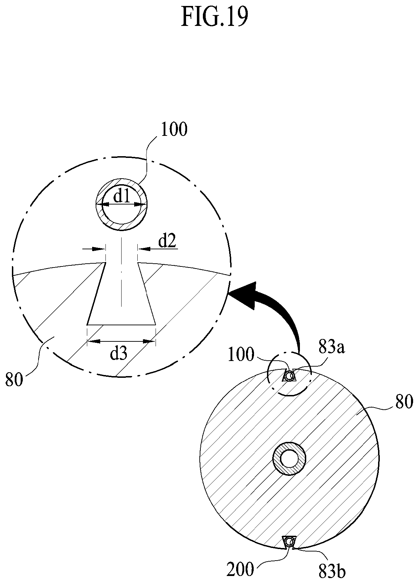

FIG. 19 is a view illustrating a guide member 80 of a stent insertion apparatus according to a second embodiment of the present invention.

In the case of the second embodiment of the present invention illustrated in FIG. 19, a fixing part of a guide member 80 includes insertion holes 83a and 83b like the above-described first embodiment. However, in the case of the present embodiment, widths of the insertion holes 83a and 83b gradually increase as depths thereof increase unlike the above-described first embodiment.

That is, in the case of the present embodiment, upper widths d₂ of the insertion holes 83a and 83b are smaller than lower widths d₃ thereof, and particularly, the upper widths d₂ of the insertion holes 83a and 83b are less than diameters d₁ of guide tubes 100 and 200.

Accordingly, the guide tubes 100 and 200 may not be easily drawn upward from the insertion holes 83a and 83b in a state in which the guide tubes 100 and 200 are inserted into the insertion holes 83a and 83b. That is, the present invention may decrease a possibility that the guide tubes 100 and 200 are drawn from the insertion holes 83a and 83b during an operation process.

In addition, the guide tubes 100 and 200 may be deformed due to expansion of a first stent 90 (see FIG. 9) caused by an elastic force thereof and drawn from the insertion holes 83a and 83b during a following operation.

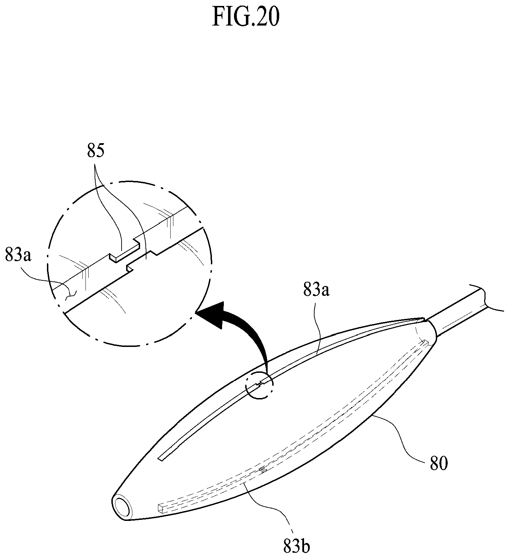

FIG. 20 is a view illustrating a guide member 80 of a stent insertion apparatus according to a third embodiment of the present invention.

In the case of the third embodiment of the present invention illustrated in FIG. 20, a pair of fixed protrusions 85 spaced by a distance less than a diameter of each of guide tubes 100 and 200 are formed at both sides of upper portions of each of insertion holes 83a and 83b.

The fixed protrusions 85 serve to fix the guide tubes 100 and 200 not to be easily drawn upward from the insertion holes 83a and 83b like the above-described second embodiment.

In addition, in the present embodiment, the guide tubes 100 and 200 are also deformed due to expansion of a first stent 90 (see FIG. 9) caused by an elastic force thereof, and may be drawn from the insertion holes 83a and 83b through a gap between the pair of fixed protrusions 85.

In addition, the fixed protrusions 85 are provided at only one position in the case of the present embodiment but may also be provided at a plurality of positions unlike the above-described case, and the positions may be variously dispersed.

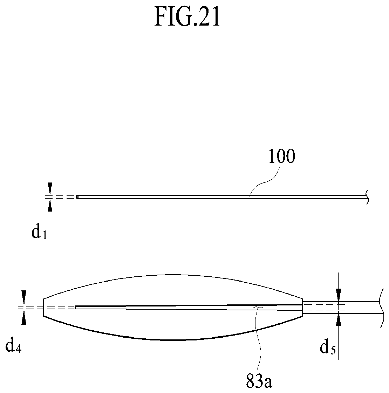

FIG. 21 is a view illustrating a guide member 80 of a stent insertion apparatus according to a fourth embodiment of the present invention.

In the case of the fourth embodiment of the present invention illustrated in FIG. 21, an insertion hole 83a is tapered forward. Here, a width d₄ of a leading end of the insertion hole 83a may be less than a diameter d₁ of a guide tube 100 inserted into the insertion hole 83a, and a width d₅ of a rear end of the insertion hole 83a may be greater than the diameter d₁ of the guide tube 100.

Accordingly, since the guide tube 100 may be fixed in a state in which the guide tube 100 is inserted into a leading end portion of the insertion hole 83a, and the guide tube 100 is formed to be freely drawn from a rear end portion of the insertion hole 83a, the guide tube 100 may be easily separated from the insertion hole 83a according to expansion of a first stent during a following operation of installing a first stent.

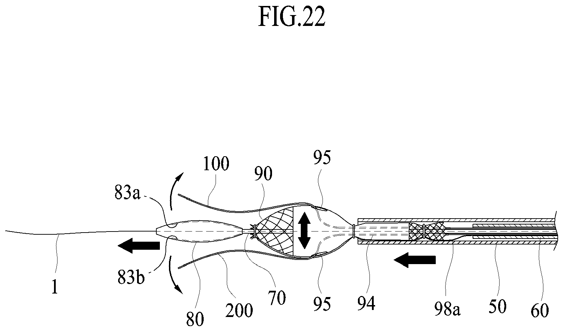

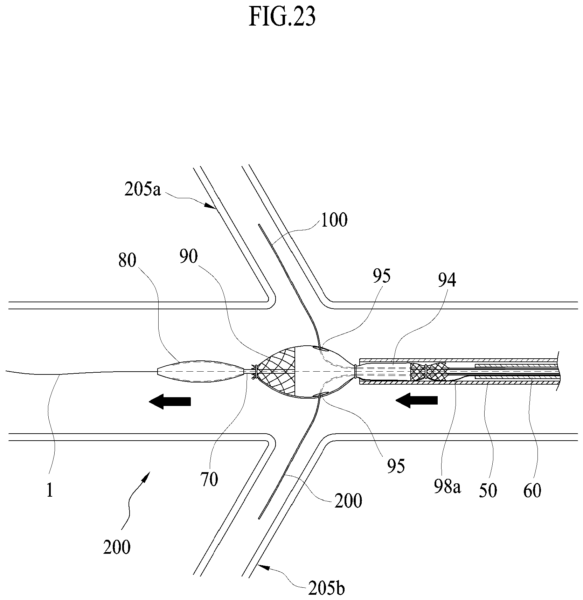

FIGS. 22 and 23 are views illustrating the guide tubes 100 and 200 inserted into branched areas of stenosis during an operation process using the stent insertion apparatus according to the fourth embodiment of the present invention.

An operation process illustrated in FIGS. 22 and 23 is a process in which the guide tubes 100 and 200 are drawn corresponding to the process illustrated in FIGS. 9 and 10 in the operation process of the above-described first embodiment. In the case of the above-described first embodiment, the operation is performed in the state in which the guide tubes 100 and 200 are inserted into second guide wires 2 and 3 in advance, but in the case of the present embodiment, the operation is performed in a state in which the second guide wires 2 and 3 are not inserted into the guide tubes 100 and 200 in advance.

In this case, after a first stent 90 is completely expanded, the second guide wires 2 and 3 may be pushed to be inserted into the guide tubes 100 and 200 right before second stents are installed, and the second guide wires 2 and 3 may be positioned at renal arteries 305a and 305b along the guide tubes 100 and 200.

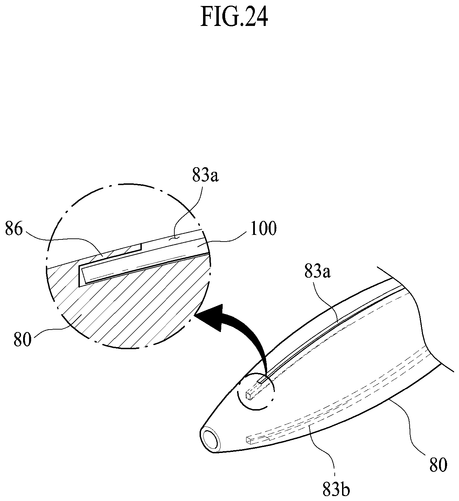

In addition, FIG. 24 is a view illustrating a guide member 80 of the stent insertion apparatus according to a fifth embodiment of the present invention.

In the case of the fifth embodiment of the present invention illustrated in FIG. 24, insertion holes 83a and 83b include covers 86 which cover partial areas of the insertion holes 83a and 83b.

The covers 86 may also serve to fix guide tubes 100 and 200 such that the tubes 100 and 200 are not drawn upward from the insertion holes 83a and 83b like the above-described embodiments and prevent the guide tubes 100 and 200 from moving backward due to friction with an inner wall of a blood vessel while the guide member 80 moves.

That is, in the case of the present embodiment, since the covers 86 prevent end portions of the guide tubes 100 and 200 from being exposed, the covers 86 may prevent the end portions of the guide tubes 100 and 200 from coming into direct contact with an inner wall of an internal organ such as the blood vessel and also prevent damage to the inner organs.

The guide member 80 according to the present embodiment has the above described shape because an operation is performed in a state in which second guide wires are not inserted into the guide tubes 100 and 200 in advance in the case of the present embodiment as described above.

In addition, in the case of the present embodiment, the cover 86 is provided at one position to shield an end portion of each of the guide tubes 100 and 200, but the cover 86 may be provided at a plurality of positions like the above-described fixed protrusion 85, and the positions may also be variously dispersed.

As described above, the present invention provides the fixing part to which the guide tubes 100 and 200 for installing the second stents are provided in the guide member 80, and a variety of shape thereof may be provided in addition to those of the above-described embodiments.

For example, as a lateral cross sectional area of at least a part of each of the insertion holes 83a and 83b may be less than that of each of the guide tubes 100 and 200, the guide tubes 100 and 200 may be fixed into the insertion holes 83a and 83b through a forcible insertion method.

As described above, the exemplary embodiments of the invention have been described, and it will be clear to those skilled in the art that the present invention may be made in different specific forms in addition to the above-described embodiments without departing from the objectives or the range thereof. Therefore, the above-described embodiments should not be understood as limitations but should be understood as examples, and accordingly, the present invention may not be limited to the above-described descriptions but may also be changed within the scope of the appended claims.

## Claims

1. A stent insertion apparatus comprising
a first stent (90),
an artificial blood vessel (94) surrounding an outer side of the first stent (90), and
a guide member (80) configured to guide movement of the first stent (90) inserted into a human body along a first guide wire (1) inserted into a main area of stenosis to be installed in the main area of stenosis,
wherein the guide member (80) includes a fixing part configured to fix leading end portions of guide tubes (100, 200) into which second guide wires (2, 3) for installing second stents (190) are inserted, the second stents (190) being inserted into branched areas of stenosis, which branch from the main area of stenosis, to be installed in the branched area of stenosis, and
wherein through holes (95) are formed in the artificial blood vessel (94) and the guide tubes (100, 200) pass through the through holes (95) to be fixed.

2. The stent insertion apparatus of claim 1, wherein the fixing part includes insertion holes (83a, 83b) formed in a longitudinal direction of the guide member (80) such that the leading end portions of the guide tubes (100, 200) are inserted into the insertion holes (83a, 83b).

## Patentansprüche

1. Stent-Einsetzvorrichtung, die Folgendes umfasst:
einen ersten Stent (90),
ein künstliches Blutgefäß (94), das eine Außenseite des ersten Stents (90) umgibt, und
ein Führungselement (80), das konfiguriert ist, eine Bewegung des ersten Stents (90), der in einen menschlichen Körper eingesetzt wird, längs eines ersten Führungsdrahts (1) zu führen, der in einen Hauptbereich einer Stenose eingesetzt wird, um in den Hauptbereich der Stenose eingesetzt zu werden,
wobei das Führungselement (80) ein Befestigungsteil umfasst, das konfiguriert ist, führende Endabschnitte der Führungsschläuche (100, 200) zu befestigen, in die zweite Führungsdrähte (2, 3) zum Installieren von zweiten Stents (190) eingesetzt sind, wobei die zweiten Stents (190) in verzweigten Bereichen der Stenose eingesetzt werden, die vom Hauptbereich der Stenose abzweigen, damit sie in dem verzweigten Bereich der Stenose installiert werden, und
wobei Durchgangslöcher (95) im künstlichen Blutgefäß (94) ausgebildet sind und die Führungsschläuche (100, 200) durch die Durchgangslöcher (95) verlaufen, so dass sie befestigt sind.

2. Stent-Einsetzvorrichtung nach Anspruch 1, wobei das Befestigungsteil Einsetzlöcher (83a, 83b) aufweist, die in Längsrichtung des Führungselements (80) ausgebildet sind, so dass die führenden Endabschnitte der Führungsschläuche (100, 200) in die Einsetzlöcher (83a, 83b) eingesetzt werden.

## Revendications

1. Appareil d'insertion d'endoprothèse comportant :
une première endoprothèse (90),
un vaisseau sanguin artificiel (94) entourant un côté extérieur de la première endoprothèse (90), et
un élément de guidage (80) configuré pour guider un mouvement de la première endoprothèse (90) insérée dans un corps humain le long d'un premier fil-guide (1) inséré dans une zone de sténose principale à installer dans la zone de sténose principale,
dans lequel l'élément de guidage (80) inclut une partie de fixation configurée pour fixer des portions d'extrémité avant de tubes de guidage (100, 200) dans lesquels sont insérés des seconds fils-guide (2, 3) destinés à installer des secondes endoprothèses (190), les secondes endoprothèses (190) étant insérées dans des zones de sténose ramifiées, qui se ramifient à partir de la zone de sténose principale, à installer dans la zone de sténose ramifiée, et
dans lequel des trous traversants (95) sont formés dans le vaisseau sanguin artificiel (94) et les tubes de guidage (100, 200) passent à travers les trous traversants (95) pour être fixés.

2. Appareil d'insertion d'endoprothèse selon la revendication 1, dans lequel la partie de fixation inclut des trous d'insertion (83a, 83b) formés dans une direction longitudinale de l'élément de guidage (80) de telle sorte que les portions d'extrémité avant des tubes de guidage (100, 200) sont insérées dans les trous d'insertion (83a, 83b).
